Europäisches Patentamt

European Patent Office

Office européen des brevets

(11) Publication number: **0 086 092**
**B1**

## (12) EUROPEAN PATENT SPECIFICATION

(45) Date of publication of patent specification: 28.05.86

(21) Application number: **83300561.4**

(22) Date of filing: **04.02.83**

(51) Int. Cl.⁴: **A 61 K 37/02** // (A61K37/02, 31:20)

(54) Antifungal compositions.

(30) Priority: **08.02.82 US 346798**
**22.11.82 US 443769**

(43) Date of publication of application:
**17.08.83 Bulletin 83/33**

(45) Publication of the grant of the patent:
**28.05.86 Bulletin 86/22**

(84) Designated Contracting States:
**AT BE CH DE FR GB IT LI LU NL SE**

(56) References cited:
**DE-A-2 803 584**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Mehta, Rajanikant Jagannath**
**684 General Scott Road**
**King of Prussia Pennsylvania 19406 (US)**

(74) Representative: **Waters, David Martin, Dr. et al**
**Smith Kline & French Laboratories Ltd. Patent Department Mundells**
**Welwyn Garden City Hertfordshire AL7 1EY (GB)**

Courier Press, Leamington Spa, England.

EP 0 086 092 B1

## Description

This invention relates to compositions containing aculeacin A and a fatty acid selected from the group consisting of oleic acid, linoleic acid, linolenic acid, lauric acid, palmitoleic acid, arachidonic acid, and palmitelaidic acid which are useful as antifungal agents.

Aculeacin A is a known antifungal antibiotic disclosed and described in the Journal of Antibiotics 30(4), pp. 297—313 (1977).

It has now been found that surprisingly the intrinsic activity of aculeacin A against fungi, such as *Candida albicans* (and especially the strains B 311, BC 759 and B 3153A, maintained in the Smith Kline & French Laboratories culture collection) is increased by the presence of the fatty acids listed.

According to the present invention there is provided a pharmaceutical or agricultural composition having antifungal activity comprising an effective amount of aculeacin A and an amount of a fatty acid from the group consisting of oleic acid, linoleic acid, linolenic acid, lauric acid, palmitoleic acid, arachidonic acid and palmitelaidic acid sufficient to potentiate the antifungal activity of aculeacin A.

According to the present invention there are also provided:

a process for preparing said composition which comprises mixing aculeacin A and the fatty acid;

aculeacin A and a fatty acid from the group consisting of oleic acid, linoleic acid, linolenic acid, lauric acid, palmitoleic acid, arachidonic acid and palmitelaidic acid for use co-operatively in the treatment of fungal infections; and

a method of treating fungal growth in cultivated land which comprises administering aculeacin A and a fatty acid from oleic acid, linoleic acid, linolenic acid, lauric acid, palmitoleic acid, arachidonic acid and palmitelaidic acid to land on which fungi is growing.

Examples of compositions within this invention are compositions containing from 5 to 3500 parts by weight of oleic acid and one part by weight of aculeacin A, provided that the minimum amount of aculeacin A is 0.039 µg. Particular compositions are those containing from 10 to 350 parts by weight of oleic acid and one part by weight of aculeacin A. Specific compositions of this invention contain at least 0.4 µg of aculeacin A and from 50 to 3500 parts by weight of oleic acid for each part by weight of aculeacin A.

A further group of compositions of this invention contain at least 0.4 µg of aculeacin A and from 5 to 360 parts by weight of linoleic acid for each part by weight of aculeacin A. In particular the compositions within this group contain at least 0.625 µg of aculeacin A and from 40 to 360 parts by weight of linoleic acid and one part by weight of aculeacin A.

A further group of compositions within the scope of this invention contain between at least 0.4 µg of aculeacin A and from 5 to 180 parts by weight of linolenic acid and one part by weight of aculeacin A. Specifically, the compositions of this group contain at least 0.625 µg of aculeacin A and from 40 to 180 parts by weight of linolenic acid and one part by weight of aculeacin A.

Further examples of compositions of this invention are compositions containing at least 1.0 µg of aculeacin A and from 250 to 1000 parts by weight of arachidonic acid or from 500 to 2000 parts by weight of palmitelaidic acid.

The antifungal activity of aculeacin A and the compositions of aculeacin A and fatty acids was measured by disc diffusion. Potentiation of antifungal activity was examined by three different methods employing: (1) a single concentration of aculeacin A and a single concentration of known fatty acids; (2) various concentrations of aculeacin A with a single concentration of individual fatty acids; and (3) various concentrations of fatty acids with a single concentration of aculeacin A. The antifungal activities of aculeacin A and the fatty acids were separately checked as controls. The fatty acids, per se, employed in the compositions of this invention do not exhibit antifungal activity at the concentrations tested.

The effects of a number of fatty acids on the antifungal activity of aculeacin A were determined by utilizing seeded plates of *Candida albicans* B 311 in yeast nitrogen base (YNB) agar (Difco) with glucose as the carbon source. A composition containing one unit by weight of aculeacin A and 5 units of fatty acid or the sodium salt thereof was spotted on the seeded plates and the size of the zone of inhibition after 16—18 hours incubation at 28—37°C was measured. The results of the above test are shown in Table I.

**0 086 092**

TABLE I

| Fatty acid | Zone size (mm) |
|---|---|
| None (control) | 15 |
| Oleic acid (sodium salt) | 32 |
| Palmitoleic acid | 26 |
| Lauric acid | 22 |
| Linoleic acid | 23 |
| Linolenic acid | 22 |
| Stearic acid | 15 |
| Palmitic acid | 15 |
| Myristic acid | 16 |
| Decanoic acid | 17 |
| Caprylic acid | 16 |

Aculeacin A and fatty acid compositions of Table I which afford an increase of 3 millimeters over aculeacin A demonstrated sufficient increase in the intrinsic activity of aculeacin A to be deemed synergistic.

Additional fatty acids were tested to determine if they increased the intrinsic activity of aculeacin A against *C. albicans* B 311 utilizing seeded plates of YNB agar (Difco) with lysine as the carbon source and seeded plates of Sabouarud dextrose agar (Difco). Compositions containing one µg of aculeacin A and various amounts of the fatty acid was spotted on the seeded plates and the size of the zone of inhibition after 16—18 hours incubation at 37°C was measured. The results of the above tests are shown, as well as a control, are shown in Table II.

TABLE II

| Aculeacin A (µg) | Fatty acid (µg) | Zone size (mm) | |
|---|---|---|---|
| | | YNB Agar | Sabouarud agar |
| 1 | None (control) | 15 | 13 |
| 1 | None (control) | 16 | 15 |
| 0 (control) | Erucic acid 1000 | 0 | 0 |
| 1 | 1000 | 15 | Trace |
| 0 (control) | 500 | 0 | 0 |
| 1 | 500 | 17 | 17 |
| 1 | 500 | 15 | 17 |
| 0 (control) | 250 | 0 | 0 |

3

TABLE II (continued)

| Aculeacin A (μg) | Vaccinic acid | Zone size (mm) | |
| --- | --- | --- | --- |
| | | YNB Agar | Sabouarud agar |
| 0 (control) | 2000 | 0 | 0 |
| 1 | 2000 | 17 | 26 |
| 0 (control) | 1000 | 0 | 0 |
| 1 | 1000 | 17 | 25 |
| 0 (control) | 500 | 0 | 0 |
| 1 | 500 | 17 | 25 |
| | Arachidonic acid | | |
| 0 (control) | 1000 | 0 | 0 |
| 1 | 1000 | 26 | 20* |
| 0 (control) | 500 | 0 | 0 |
| 1 | 500 | 20 | 20* |
| 0 (control) | 250 | 0 | 0 |
| 1 | 250 | 20 | 20* |
| | Homo-γ-linoleic acid | | |
| 0 (control | 200 | 0 | 0 |
| 1 | 200 | 17* | 15 |
| 0 (control) | 100 | 0 | 0 |
| 1 | 100 | 15* | 15 |
| 0 (control) | 50 | 0 | 0 |
| 1 | 50 | 15* | 15 |
| | Palmitelaidic acid | | |
| 0 (control) | 2000 | 0 | 0 |
| 1 | 2000 | 22 | 25 |
| 0 (control) | 1000 | 0 | 0 |
| 1 | 1000 | 20 | 32 |
| 0 (control) | 500 | 0 | 0 |
| 1 | 500 | 20 | 30 |

4

TABLE II (continued)

| Aculeacin A (µg) | α-Hydroxy lauric acid | Zone size (mm) | |
| --- | --- | --- | --- |
| | | YNB Agar | Sabouarud agar |
| 0 (control) | 1000 | Trace | 10 (hazy) |
| 1 | 1000 | 25 | 20 |
| 0 (control) | 500 | Trace | 10 (hazy) |
| 1 | 500 | 24 | 15 |
| 0 (control) | 250 | Trace | Trace |
| 1 (control) | 250 | 20 | 15 |
| Behenic acid | | | |
| 0 (control) | 1000 | 0 | 0 |
| 1 | 1000 | 15 | 20 |
| 0 (control) | 500 | 0 | 0 |
| 1 | 500 | 14 | 14 |
| 0 (control) | 250 | 0 | 0 |
| 1 | 250 | 14 | 15 |
| Eladic acid | | | |
| 0 (control) | 1000 | 0 | 0 |
| 1 | 1000 | 13 | 13 |
| 0 (control) | 500 | 0 | 0 |
| 1 | 500 | 13 | 15 |
| 0 (control) | 250 | 0 | 0 |
| 1 | 250 | 13 | 18 |
| Oleic acid sodium salt | | | |
| 0 (control) | 1000 | 0 | 0 |
| 1 | 1000 | 35 | 26 |
| 0 (control) | 500 | 0 | 0 |
| 1 | 500 | 35 | 26 |
| 0 (control) | 250 | 0 | 0 |
| 1 | 250 | 35 | 28 |

* irregular zone of inhibition.

Aculeacin A and fatty acid compositions of Table II which afforded an increase of 3 millimeters over Aculeacin A in both the YNB agar and the Sabouarud dextrose agar demonstrated a sufficient increase in the intrinsic activity of aculeacin A to be deemed synergistic.

The antifungal effects of compositions containing a single amount of oleic acid as the sodium salt while varying the amounts of aculeacin A were determined by utilizing seeded plates of Candida albicans B

5

311 in Sabouarud dextrose agar and measuring the zone of inhibition after 16—18 hours incubation at 28—37°C. The results of the above test, as well as a control, are shown in Table III.

TABLE III

| Aculeacin A (µg) | Oleic acid (µg) | Zone size (mm) |
|---|---|---|
| 2.5 | 0 | 19 |
| 1.25 | 0 | 14—16 |
| 1.25 | 12.5 | 37 |
| 0.625 | 0 | 10 |
| 0.625 | 12.5 | 35 |
| 0.312 | 0 | trace |
| 0.312 | 12.5 | 35 |
| 0.156 | 12.5 | 32 |
| 0.078 | 12.5 | 29 |
| 0.039 | 12.5 | 28 |
| 0.020 | 12.5 | trace |
| 0.000 | 12.5 | 0 |

The antifungal effects of compositions containing a single amount of aculeacin A while varying the amounts of oleic acid as the sodium salt were determined by utilizing seeded plates of *Candida albicans* strains B 311, BC 759 and B 3153A and measuring the zone of inhibition after 16—18 hours at 28—37°C. This test was also performed with linoleic acid and linolenic acid. The results of these tests are shown in Table IV-a, IV-b and IV-c.

TABLE IV-a

| Aculeacin A (µg) | Oleic acid (µg) | Zone size (mm) | | |
|---|---|---|---|---|
| | | B 311 | BC 759 | B 3153-A |
| 0.40 | 0 | 10 | 13 | 12 |
| 0.40 | 5 | 13 | 13 | 13 |
| 0.40 | 10 | 14 | 15 | 15 |
| 0.40 | 20 | 15 | 18 | 18 |
| 0.40 | 40 | 17 | 18 | 17 |
| 0.40 | 78 | 20 | 20 | 18 |
| 0.40 | 156 | 26 | 24 | 25 |
| 0.40 | 312 | 30 | 28 | 25 |
| 0.40 | 625 | 32 | 29 | 28 |
| 0.40 | 1250 | 35 | 32 | 29 |

6

# 0 086 092

## TABLE IV-b

| Aculeacin A (µg) | Linoleic acid (µg) | Zone size (mm) B 311 |
|---|---|---|
| 0.625 | 225 | 19 |
| 0 | 225 | Trace |
| 0.625 | 112.5 | 21 |
| 0 | 112.5 | 0 |
| 0.625 | 56.2 | 22 |
| 0 | 56.2 | 0 |
| 0.625 | 28.1 | 22 |
| 0 | 28.1 | 0 |
| 0.625 | 0 | 16 |

## TABLE IV-c

| Aculeacin A (µg) | Linolenic acid (µg) | Zone size (mm) B 311 |
|---|---|---|
| 0.625 | 112.5 | 19 |
| 0 | 112.5 | 0 |
| 0.625 | 56.2 | 19 |
| 0 | 56.2 | 0 |
| 0.625 | 28.1 | 20 |
| 0 | 28.1 | 0 |
| 0.625 | 0 | 16 |

The seeded plates of *Candida albicans* strains B 311, BC 759, and B 3153A were prepared by inoculating 50 ml of trypticase soy broth (BBL) with one ml of the preserved frozen culture and incubating the inoculum at 37°C for 7 hours on a rotary shaker. Two milliliters of this culture were used to inoculate one liter of Sabouarud dextrose agar (Difco) at 50°C and 15 milliliters of the resultant medium poured into 150 mm petri-dishes.

The antifungal activity of the compositions of this invention, as exemplified by the composition containing Aculeacin A and oleic acid as shown below, was demonstrated by topical treatment of *C. albicans* infections of mice. Clinical isolates of *C. albicans* were grown in Sabouarud dextrose agar and subcultured into mouse serum and incubated at 35°C. Female mice (CF-1, 4 to 6 weeks old) were shaved on the back (2 cm² area). The shaved area was cleaned with 70% alcohol and scrubbed with a wire brush. This area was then painted with the *C. albicans* in mouse serum as noted above ($10^5$—$10^6$ CFu/ml) and after drying this area was covered with sterile gauze and the infection was allowed to develop over two days. After the development of the infection, the mice were treated with aculeacin A/oleic acid composition, aculeacin A and Lotrimin® (Clotrimazole-Schering) for 5 days. The minimum inhibitory concentration (MIC) for aculeacin A for each clinical isolate of *C. albicans* was determined and that amount was employed in each experiment. Commercially available Lotrimin® cream was utilized in each experiment. As the results below demonstrate, the aculeacin A/oleic acid composition afforded a significant reduction in the infection (comparable to Lotrimin®) over aculeacin A, itself, or the control. Oleic acid was tested separately and did not inhibit the infection. Note that bacterial contamination was ruled out by microscopic examination.

7

| Experiment number | Untreated control | Lotrimin® | Aculeacin A (µg) | Aculeacin A/oleic acid (µg/µg) |
|---|---|---|---|---|
| I | 4 | 0 | 1 (0.8) | 0 (0.5/100) |
|  | 3 | 0 | 1 | 0 |
| II | 2 | 1 | 0 (1.5) | 0 (0.9/100) |
|  | 2 | 1 | 1 | 0 |
| III | 3 | 0 | 1 (5.0) | 0 (1.5/100) |
|  | 3 | 0 | 1 | 0 |
| IV | 3 | 0 | 2 (0.5) | 1 (0.5/100) |
|  | 3 | 0 | 2 | 0 |
| V | 3 | 0 | 0 (10.5) | 0 (5.0/100) |
|  | 3 | 0 | 0 | 0 |
| VI | 3 | 0 | 1 (5.0) | 1 (5.0/100) |
|  | 3 | 0 | 1 | 1 |

where
4—the most severe infection-zone of inoculation highly erythrematous with culture mainly consisting of *C. albicans*
3—severe infection with some healing
2—less severe infection
1—very little infection
0—no infection.

In view of the above described antifungal activity, antifungal amounts of the compositions of this invention may be employed as the active ingredients in pharmaceutically acceptable vehicles, such as solid, semi-solid or liquid carriers, to combat fungus growth. This composition in pharmaceutical form may be applied to the area to be treated by conventional methods, such as, dusting, spraying, brushing, smearing, impregnating or other suitable means. The instant compositions may also be employed in agricultural forms to treat fungus growth in cultivated fields.

**Claims for the Contracting States: BE, CH/LI, DE, FR, GB, IT, LU, NL, SE**

1. A pharmaceutical or agricultural composition having antifungal activity comprising an effective amount of aculeacin A and an amount of a fatty acid from the group consisting of oleic acid, linoleic acid, linolenic acid, lauric acid, palmitoleic acid, arachidonic acid and palmitelaidic acid sufficient to potentiate the antifungal activity of aculeacin A.

2. A composition as claimed in claim 1 containing at least 0.039 µg of aculeacin A and from 5 to 3500 parts by weight of oleic acid for each part by weight of aculeacin A.

3. A composition as claimed in claim 2 containing between 10 and 350 parts by weight of oleic acid.

4. A composition as claimed in claim 2 containing at least 0.4 µg of aculeacin A and at least 50 parts by weight of oleic acid.

5. A composition as claimed in claim 1 containing at least 0.4 µg of aculeacin A and from 5 to 360 parts by weight of linoleic acid or from 5 to 180 parts by weight of linolenic acid for each part by weight of aculeacin A.

6. A composition as claimed in claim 5 containing at least 0.625 µg of aculeacin A and at least 40 parts by weight of linoleic acid or linolenic acid.

7. A composition as claimed in claim 1 comprising at least 1.0 µg of aculeacin A and either from 250 to 1000 parts by weight of arachidonic acid or from 500 to 2000 parts by weight of palmitelaidic acid for each part by weight of aculeacin A.

8. A process for preparing a composition as claimed in any one of claims 1 to 7 which comprises mixing aculeacin A and the fatty acid.

9. Aculeacin A and fatty acid from the group consisting of oleic acid, linoleic acid, linolenic acid, lauric acid, palmitoleic acid, arachidonic acid and palmitelaidic acid for use co-operatively in the treatment of fungal infections.

10. A method of treating fungal growth in cultivated land which comprises administering aculeacin A and a fatty acid from oleic acid, linoleic acid, linolenic acid, lauric acid, palmitoleic acid, arachidonic acid and palmitelaidic acid to land on which fungi is growing.

# 0 086 092

**Claim for the Contracting State: AT**

A process for the preparation of an antifungal composition comprising an antifungal effective amount of aculeacin A and an amount of fatty acid from the group consisting of oleic acid, linoleic acid, linolenic acid, lauric acid, palmitoleic acid, arachidonic and palmitelaidic acid sufficient to potentiate the bioactivity of aculeacin A; which comprises mixing the required amounts of aculeacin A and fatty acid.

**Patentansprüche für die Vertragsstaaten: BE, CH/LI, DE, FR, GB, IT, LU, NL, SE**

1. Arzneimittel oder Landwirtschaftschemikalie mit Wirksamkeit gegen Pilze, umfassend eine wirksame Menge von Aculeacin A und eine zur Potentierung der Wirksamkeit gegen Pilze von Aculeacin A ausreichende Menge einer der Fettsäuren Ölsäure, Linolsäure, Linolensäure, Laurinsäure, Palmitoleinsäure, Arachidonsäure oder Palmitelaidinsäure.

2. Mittel nach Anspruch 1, enthaltend mindestens 0,039 µg Aculeacin A und 5 bis 3500 Gewichtsteile Ölsäure pro Gewichtsteil Aculeacin A.

3. Mittel nach Anspruch 2, enthaltend 10 bis 350 Gewichtsteile Ölsäure.

4. Mittel nach Anspruch 2, enthaltend mindestens 0,4 µg Aculeacin A und mindestens 50 Gewichtsteile Ölsäure.

5. Mittel nach Anspruch 1, enthaltend mindestens 0,4 µg Aculeacin A und 5 bis 360 Gewichtsteile Linolsäure oder 5 bis 180 Gewichtsteile Linolensäure pro Gewichtsteil Aculeacin A.

6. Mittel nach Anspruch 5, enthaltend mindestens 0,625 µg Aculeacin A und mindestens 40 Gewichtsteile Linolsäure oder Linolensäure.

7. Mittel nach Anspruch 1, umfassend mindestens 1,0 µg Aculeacin A und entweder 250 bis 1000 Gewichtsteile Arachidonsäure oder 500 bis 2000 Gewichtsteile Palmitelaidinsäure pro Gewichtsteil Aculeacin A.

8. Verfahren zur Herstellung eines Mittels nach einem der Ansprüche 1 bis 7 durch Mischen von Aculeacin A und der Fettsäure.

9. Aculeacin A und eine der Fettsäuren Ölsäure, Linolsäure, Linolensäure, Laurinsäure, Palmitoleinsäure, Arachidonsäure und Palmitelaidinsäure zur gemeinsamen Verwendung bei der Behandlung von Pilzinfektionen.

10. Verfahren zur Behandlung des Pilzwachstums in kultiviertem Land durch Verabreichung von Aculeacin A und einer der Fettsäuren Ölsäure, Linolsäure, Linolensäure, Laurinsäure, Palmitoleinsäure, Arachidonsäure und Palmitelaidinsäure auf das Land, auf dem die Pilze wachsen.

**Patentanspruch für den Vertragsstaat: AT**

Verfahren zur Herstellung eines Mittels gegen Pilze aus einer gegen Pilze wirksamen Menge von Aculeacin A und einer zur Potentierung der Bioaktivität von Aculeacin A ausreichenden Menge einer der Fettsäuren Ölsäure, Linolsäure, Linolensäure, Laurinsäure, Palmitoleinsäure, Arachidonsäure oder Palmitelaidinsäure, gekennzeichnet durch Vermischen der erforderlichen Mengen Aculeacin A und Fettsäure.

**Revendications pour les Etats Contractants: BE, CH, DE, FR, GB, IT, LI, LU, NL, SE**

1. Une composition pharmaceutique ou agricole ayant une activité antifongique et comprenant une quantité efficace d'aculéacine A et une quantité d'un acide gras en provenance du groupe composé de l'acide oléique, l'acide linoléique, l'acide linolénique, l'acide laurique, l'acide palmitoléique, l'acide arachidonique et l'acide poalmitélaidique, suffisante pour potentialiser l'activité antifongique de l'aculéacine A.

2. Une composition comme indiquée dans la revendication 1 contenant au moins 0,039 µg d'aculéacine A et entre 5 et 3500 parts par poids d'acide oléique pour chaque part par poids d'aculéacine A.

3. Une composition comme indiqué dans la revendication 2 contenant entre 10 et 350 parts par poids d'acide oléique.

4. Une composition comme indiqué dans la revendication 2 contenant au moins 0,4 µg d'aculéacine A et au moins 50 parts par poids d'acide oléique.

5. Une composition comme indiqué dans la revendication 1 contenant au moins 0,4 µg d'aculéacine A et entre 5 et 350 parts par poids d'acide linoléique ou bien entre 5 et 180 parts par poids d'acide linolénique pour chaque part par poids d'aculéacine A.

6. Une composition comme indiqué dans la revendication 5 contenant au moins 0,625 µg d'aculéacine A et au moins 40 parts par poids d'acide linoléique ou d'acide linolénique.

7. Une composition comme indiqué dans la revendication 1 comprenant au moins 1,0 µg d'aculéacine A et soit entre 250 et 1000 parts par poids d'acide arachidonique ou bien entre 500 et 2000 parts par poids d'acide palmiélaidique pour chaque part par poids d'aculéacine A.

8. Un procédé pour la préparation d'une composition comme indiqué dans n'importe laquelle des revendications de 1 à 7 qui comprend le mélange de l'aculéacine A et de l'acide gras.

9

9. L'aculéacine A et un acide gras en provenance du groupe comprenant l'acide oléique, l'acide linoléique, l'acide linolénique, l'acide laurique, l'acide palmitoléique, l'acide arachidonique et l'acide palmitélaidique pour l'emploi aux fins d'action simultanée dans le traitement des infections fongiques.

10. Une méthode de traitement de la croissance fongique dans les terres cultivées qui comprend l'administration de l'aculéacine A et d'un acide gras en provenance de l'acide oléique, l'acide linoléique, l'acide linolénique, l'acide laurique, l'acide palmitoléique, l'acide arachidonique et l'acide palmitélaidique, à des terres sur lesquelles poussent les fongi.

**Revendications pour l'Etat Contractant: AT**

Un procédé pour la préparation d'une composition antifongique qui comprend une quantité efficace antifongique d'aculéacine A et une quantité d'acide gras en provenance du groupe comprenant l'acide oléique, l'acide linoléique, l'acide linolénique, l'acide laurique, l'acide palmitoléique, l'acide arachidonique et l'acide palmitélaidique suffisante pour potentialiser l'activité biologique de l'aculéacine A; qui comprend le mélange en quantités requises de l'aculéacine A et d'acide gras.